(19) Europäisches Patentamt

European Patent Office

Office européen des brevets

(11) **EP 1 227 759 B1**

(12) **EUROPÄISCHE PATENTSCHRIFT**

(45) Veröffentlichungstag und Bekanntmachung des Hinweises auf die Patenterteilung:
**02.01.2004  Patentblatt 2004/01**

(51) Int Cl.⁷: **A61B 17/00**

(86) Internationale Anmeldenummer:
**PCT/DE1999/003337**

(21) Anmeldenummer: **99960790.6**

(22) Anmeldetag: **14.10.1999**

(87) Internationale Veröffentlichungsnummer:
**WO 2001/026556 (19.04.2001 Gazette 2001/16)**

(54) **DREHGELENK FÜR MEDIZINISCHE INSTRUMENTE**

ROTATION JOINT FOR MEDICAL INSTRUMENTS

ARTICULATION TOURNANTE POUR INSTRUMENTS MEDICAUX

(84) Benannte Vertragsstaaten:
**DE FR GB IT**

(43) Veröffentlichungstag der Anmeldung:
**07.08.2002  Patentblatt 2002/32**

(73) Patentinhaber: **Karl Storz GmbH & Co.**
**78532 Tuttlingen (DE)**

(72) Erfinder: **DUBROWSKIJ, Arkadij Veniaminowitsch**
**Moskau 101000 (RU)**

(74) Vertreter: **Hofmeister, Frank Horst**
**Kleiststrasse 7**
**40878 Ratingen (DE)**

(56) Entgegenhaltungen:
WO-A-99/21686          US-A- 4 641 657
US-A- 5 209 747        US-A- 5 549 637
US-A- 5 575 799        US-A- 5 772 655

**Beschreibung**

Die Erfindung betrifft ein Drehgelenk, insbesondere für medizinische Instrumente, mit einem proximalen und einem distalen Teil, die über ein Zahnradgetriebe miteinander gekoppelt und gegeneinander zwischen einer nicht ausgelenkten Ausgangsstellung und einer abgewinkelten Endstellung verstellbar sind, wobei das Zahnradgetriebe in einem zwischen dem proximalen Teil und dem distalen Teil angeordneten und diese Teile voneinander beabstandenden, im Längsschnitt trapezförmigen Zwischenteil gelagert ist, wobei die einander zugewandten und in der Ausgangsstellung an geneigten Stirnflächen des trapezförmigen Zwischenteils anliegenden Stirnflächen des distalseitigen Endes des proximalen Teils und des proximalseitigen Endes des distalen Teils unter dem gleichen Winkel zur Längsachse aufeinander zu geneigt sind und alle drei Teile zumindest einen durchgehenden hohlen Zentralkanal aufweisen.

Aus der Medizintechnik sind verschiedene Konstruktionen bekannt, um den Abweichungswinkel der Arbeitsteile eines medizinischen Instruments von der Längsachse des Gehäuses zu verändern. Diese bekannten Drehgelenke weisen häufig die Nachteile auf, daß die Verstellung der Winkellage nicht ferngesteuert, d.h. von einer Position fern ab des eigentlichen Gelenks betätigt werden kann, die eingestellte Winkellage nicht fixiert werden kann und/oder die Gelenke keinen hohlen Zentralkanal aufweisen, um beispielsweise die Arbeitsteile am distalen Ende des Instruments über einen Seilzug zu betätigen.

Ein Drehgelenk der eingangs genannten Art ist beispielsweise aus der US-PS 4 641 657 bekannt. Dieses bekannte Drehgelenk weist den Nachteil auf, daß das Verstellen der Winkellage der einzelnen Gelenkteile zueinander über das Verdrehen des Zwischenteils erfolgt, weshalb eine Fembetätigung des Gelenks nicht möglich ist Weiterhin weist die vorbekannte Konstruktion keine Mittel auf, um das Gelenk in der ausgelenkten Position zu fixieren.

Gelenk entfernten Position betätigbar ist und die eingestellte Winkellage der Gelenkteile zueinander fixierbar ist.

Das erfindungsgemäße Drehgelenk ist dadurch gekennzeichnet, daß der proximale Teil aus zwei koaxial ineinander angeordneten und unabhängig voneinander gegeneinander um ihre Längsachsen verdrehbaren Rohren besteht, wobei zumindest das äußere Rohr proximalseitig mit einer Handhabe verbunden ist und an der distalseitigen Stirnfläche des äußeren Rohres ein Steuerzapfen angeordnet ist, der in eine entsprechende Ausnehmung des Zwischenteils eingreift.

Über die mit dem äußeren Rohr des proximalen Teils verbundene Handhabe und den an der distalseitigen Stirnfläche des äußeren Rohres ein angeordneten Steuerzapfen, der in eine entsprechende Ausnehmung des Zwischenteils eingreift ist es möglich, die Winkelstellung des Drehgelenks durch unabhängig voneinander stattfindendes Verdrehen entweder des inneren Rohres oder des äußeren Rohres zu verändern. Dabei ist es möglich, das Gelenk durch Verdrehen des äußeren Rohres zu verstellen, ohne daß eine direkte Verstellung des das Zahnradgetriebe aufweisenden Zwischenteils erforderlich ist. Neben dem ferngelenkten Verstellen des Gelenks ermöglicht die Handhabe das Fixieren des Gelenks in der jeweils ausgelenkten Stellung. Als weiterer Vorteil der erfindungsgemäßen Konstruktion ist hervorzuheben, daß das Gelenk durch die koaxiale Anordnung der beiden das proximale Teil bildenden Rohre aufeinander sehr steif ist.

Die gleichbleibende Steifigkeit des um den gewünschten Winkel drehbaren Drehgelenks wird dabei dadurch erzielt, daß die einander zugewandten und in der Ausgangsstellung an Stirnflächen des Zwischenteils anliegenden Stirnflächen des distalseitigen Endes des proximalen Teils und des proximalseitigen Endes distalen Teils unter dem gleichen Winkel zur Längsachse aufeinander zu geneigt sind.

Weiterhin wird mit der Erfindung vorgeschlagen, daß das innere Rohr distalseitig mit einem Zahnrad des Zahnradgetriebes versehen ist.

Gemäß einer bevorzugten Ausführungsform der Erfindung wird vorgeschlagen, daß das Zahnradgetriebe aus wenigstens zwei miteinander in Eingriff stehenden Kegelrädern besteht, wobei ein Kegelrad mit dem distalseitigen Ende des inneren Rohres und ein anderes Kegelrad mit dem proximalseitigen Ende des distalen Teils verbunden ist. Bei der Verwendung von zwei miteinander in Eingriff stehenden Kegelrädern ist es möglich, den proximalen und den distalen Teil des Drehgelenks um jeweils den gleichen Winkel gegenüber der Längsachse auszulenken.

Um einen besonders flachen Krümmungsbogen zwischen dem proximalen Teil und dem distalen Teil zu ermöglichen, was wiederum die Gefahr von Quetschungen der durch den hohlen Zentralkanal geführten Betätigungsmittel reduziert, wird gemäß einer zweiten Ausführungsform der Erfindung vorgeschlagen, daß die beiden mit dem proximalen und distalen Teil verbundenen Kegelräder über ein im Zwischenteil gelagertes Kegelrad miteinander verbunden sind. Das Zwischenstück ist bei dieser Variante um den Durchmesser des zusätzlichen Kegelrades verlängert, woraus ein größerer Radius des Krümmungsbogens resultiert.

Schließlich wird mit der Erfindung vorgeschlagen, daß sich der maximale Auslenkungswinkel des solchermaßen ausgestalteten Drehgelenks zwischen der Ausgangsstellung und der Endstellung aus der Gleichung 4 x (90° - $\alpha$) ergibt. Für einen maximalen Auslenkungswinkel von 90° ergibt sich somit für $\alpha$ ein Wert von 67,5° und für einen maximalen Auslenkungswinkel von 180° ein Wert von 45° für $\alpha$.

Weitere Merkmale und Vorteile der Erfindung ergeben sich aus der nachfolgenden Beschreibung der zugehörigen Zeichnung, in der zwei Ausführungsfor-

men eines erfindungsgemäßen Drehgelenks beispiel-haft dargestellt sind. In der Zeichnung zeigt:

Fig. 1 eine teilweise geschnittene Seitenansicht einer ersten Ausführungsform eines erfindungsgemäßen Drehgelenks in der nicht ausgelenkten Ausgangsstellung;

Fig. 2 eine Seitenansicht des Drehgelenks gemäß Fig. 1 in der abgewinkelten Endstellung und

Fig. 3 eine teilweise geschnittene Seitenansicht einer zweiten Ausführungsform eines erfindungsgemäßen Drehgelenks in der nicht ausgelenkten Ausgangsstellung.

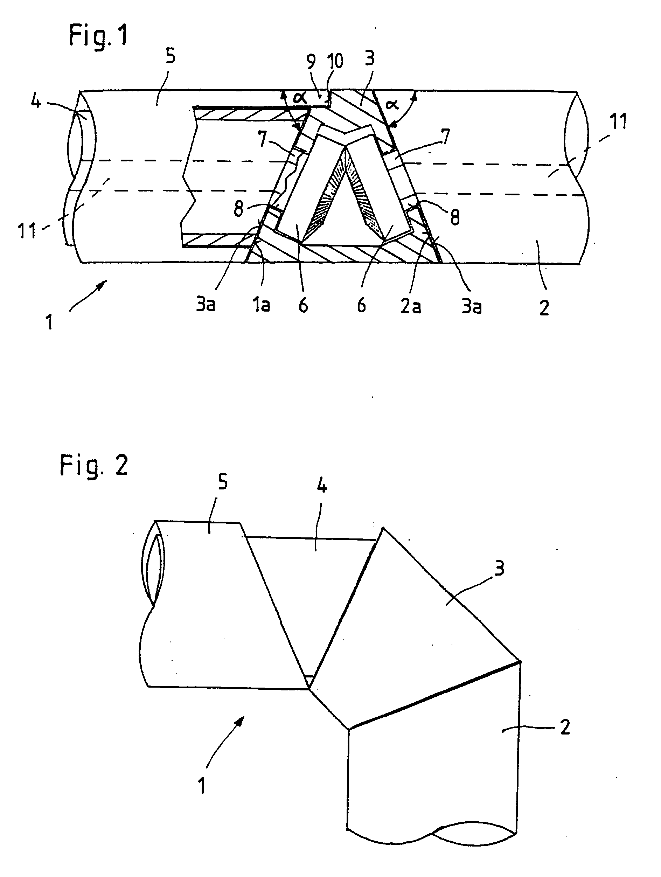

Die in den Abbildungen ausschnittweise dargestellten Drehgelenke bestehen im wesentlichen aus einem proximalen Teil 1, einem distalen Teil 2 und einem zwischen dem proximalen Teil 1 und dem distalen Teil 2 angeordneten Zwischenteil 3. Wie aus den Abbildungen Fig. 1 und 2 ersichtlich, besteht der proximale Teil 1 aus zwei koaxial ineinander angeordneten Rohren, nämlich einem inneren Rohr 4 und einem äußeren Rohr 5.

Solche Drehgelenke dienen bei medizinischen Instrumenten dazu, einen Abweichungswinkel der Arbeitsteile eines medizinischen Instruments von der Längsachse des Instrumentenschaftes zu ermöglichen. Um eine Verstellung des Drehgelenks von einer gelenkfernen Position aus zu ermöglichen, wie dies beispielsweise bei endochirurgischen Instrumenten notwendig ist, weist zumindest das äußere Rohr 5 proximalseitig eine in den Abbildungen nicht dargestellte Handhabe auf, über die das äußere Rohr 5 gegenüber dem inneren Rohr 4 um die Längsachse der beiden Rohre 4, 5 verdrehbar ist. Die Handhabe dient weiterhin dazu, das Drehgelenk in der jeweils ausgelenkten Stellung zu fixieren.

Bei der in Fig. 1 dargestellten Ausführungsform eines Drehgelenks sind der proximale Teil 1 und der distale Teil 2 über ein aus zwei Kegelrädern 6 bestehendes Zahnradgetriebe miteinander gekoppelt und gegeneinander verdrehbar.

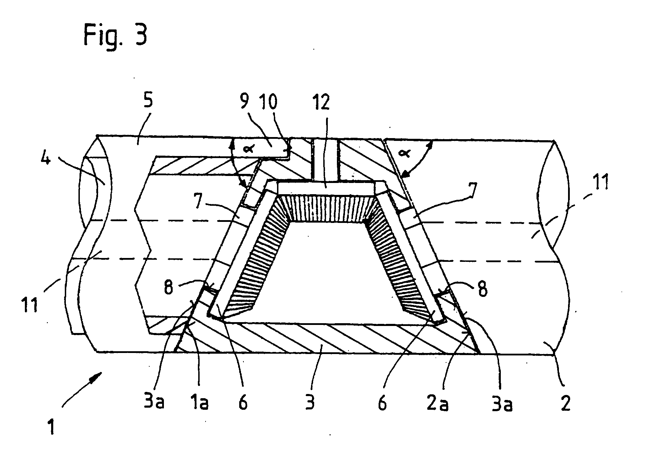

Wie aus den in den Abbildungen Fig. 1 und 3 dargestellten Ausgangsstellungen ersichtlich, in den die drei Teile 1, 2 und 3 nicht gegeneinander ausgelenkt sind, sind die einander zugewandten und an den Stirnflächen 3a des Zwischenteils 3 anliegenden Stirnflächen 1 a und 2a des proximalen Teils 1 und des distalen Teils 2 unter dem gleichen Winkel $\alpha$ zur Längsachse des Drehgelenks aufeinander zu geneigt. Die das Zahnradgetriebe bildenden Kegelräder 6 sind über senkrecht auf den Stirnflächen 1a des distalseitigen Endes des inneren Rohres 4 und des proximalseitigen Endes des distalen Teils 2 angeordnete Achsstummel 7 mit dem proximalen Teil 1 und dem distalen Teil 2 verbunden. In den Stirnflächen 3a des Zwischenteils 3 sind dem Durchmesser der Achsstummel 7 entsprechende Durchbrüche 8 so ausgebildet, daß die Ränder der Durchbrüche 8 die Kegelräder 6 hintergreifen und so der Gesamtkonstruktion die ausreichende Steifigkeit verleihen.

Zum Verstellen des Drehgelenks über die mit dem proximalseitigen Ende zumindest des äußeren Rohres 5 verbundene Handhabe ist an einer Stelle der distalseitigen Stirnfläche 1a des äußeren Rohres 5 ein Steuerzapfen 9 angeformt, der in eine entsprechende Ausnehmung 10 des Zwischenteils 3 eingreift. Ein Verdrehen des äußeren Rohres 5 bei gleichzeitigem Stillstand des inneren Rohres 4 bewirkt, daß über den Steuerzapfen 9 das Zwischenteil 3 bis in die in Fig. 2 dargestellte Endstellung verdreht werden kann. Die gekoppelten Stirnflächen 1a - 3a und 3a - 2a ermöglichen die Drehung des Drehgelenks um den gewünschten Winkel, wobei die Steifigkeit des Drehgelenks erhalten bleibt.

Die gleiche Verstellung des Drehgelenks in die in Fig. 2 dargestellte Endstellung kann durch Verdrehen des inneren Rohres 4 bei gleichzeitigem Stillstand des äußeren Rohres 5 bewirkt werden, wobei die exakte Verstellung der Teile 1, 2 und 3 des Drehgelenks über die in Eingriff miteinander stehenden Kegelräder 6 erfolgt.

Das Drehgelenk zeichnet sich dadurch aus, daß Krümmungsbogen zwischen dem proximalen Teil 1 und dem distalen Teil 2 in der maximal ausgelenkten Endstellung einen großen Radius aufweist, damit durch einen durch alle drei Teile 1, 2 und 3 verlaufenden hohlen Zentralkanal 11 geführte Betätigungsmittel, wie beispielsweise ein Seil, ein flexibler Schlauch oder Strom- oder Lichtleiter für ein medizinisches Arbeitsteil, nicht geknickt oder gequetscht werden.

Bei der in Fig. 3 dargestellten Ausführungsform besteht das Zahnradgetriebe aus drei Kegelrädern, wobei die beiden mit dem proximalen Teil 1 und distalen Teil 2 verbundenen Kegelräder 6 über ein in dem Zwischenteil 3 gelagertes Kegelrad 12 miteinander verbunden sind. Da sich bei dieser Ausgestaltungsform das Zwischenteil 3 um den Durchmesser des dritten Kegelrades 12 verlängert, erlaubt diese Bauform einen noch flacheren Krümmungsbogen.

Der maximale Auslenkungswinkel zwischen der Ausgangsstellung und der Endstellung ergibt sich aus der Gleichung 4 x (90° - $\alpha$). Um also einen maximalen Auslenkungswinkel von 90° erhalten zu wollen muß der Winkel $\alpha$, um den die Stirnflächen 1 a und 2a von der Längsachse fort aufeinander zu geneigt sind, 67,5° betragen. Bei einem Wert von 45° für den Winkel $\alpha$ ergibt sich ein Auslenkungswinkel von 180°.

Wie aus Fig. 1 und 2 ersichtlich, ist der Drehwinkel des äußeren Rohres 5 mit dem Steuerzapfen 9 unbegrenzt 360° in beliebige Richtung, wobei der maximale Auslenkungswinkel bei einer Drehung um 180° erzielt wird.

**Bezugszeichenliste**

| | |
|---|---|
| 1 | proximaler Teil |
| 1 a | Stirnfläche |
| 2 | distaler Teil |
| 2a | Stirnfläche |
| 3 | Zwischenteil |
| 3a | Stirnfläche |
| 4 | inneres Rohr |
| 5 | äußeres Rohr |
| 6 | Kegelrad |
| 7 | Achsstummel |
| 8 | Durchbruch |
| 9 | Steuerzapfen |
| 10 | Ausnehmung |
| 11 | hohler Zentralkanal |
| 12 | Kegelrad |
| α | Winkel |

**Patentansprüche**

1. Drehgelenk, insbesondere für medizinische Instrumente, mit einem proximalen (1) und einem distalen Teil (2), die über ein Zahnradgetriebe miteinander gekoppelt und gegeneinander zwischen einer nicht ausgelenkten Ausgangsstellung und einer abgewinkelten Endstellung verstellbar sind, wobei das Zahnradgetriebe in einem zwischen dem proximalen Teil (1) und dem distalen Teil (2) angeordneten und diese Teile (1 und 2) voneinander beabstandenden, im Längsschnitt trapezförmigen Zwischenteil (3) gelagert ist, wobei die einander zugewandten und in der Ausgangsstellung an geneigten Stirnflächen (3a) des trapezförmigen Zwischenteils (3) anliegenden Stirnflächen des distalseitigen Endes (1a) des proximalen Teils (1) und des proximalseitigen Endes (2a) des distalen Teils (2) unter dem gleichen Winkel (α) zur Längsachse aufeinander zu geneigt sind und alle drei Teile (1, 2 und 3) zumindest einen durchgehenden hohlen Zentralkanal (11) aufweisen, **dadurch gekennzeichnet, daß** der proximale Teil (1) aus zwei koaxial ineinander angeordneten und unabhängig voneinander gegeneinander um ihre Längsachsen verdrehbaren Rohren (4, 5) besteht, wobei zumindest das äußere Rohr (5) proximalseitig mit einer Handhabe verbunden ist und an der distalseitigen Stirnfläche (1 a) des äußeren Rohres (5) ein Steuerzapfen (9) angeordnet ist, der in eine entsprechende Ausnehmung (10) des Zwischenteils (3) eingreift.

2. Drehgelenk nach er Anspruch 1, **dadurch gekennzeichnet, daß** das innere Rohr (4) distalseitig mit einem Zahnrad des Zahnradgetriebes versehen ist.

3. Drehgelenk nach Anspruch 1 oder 2, **dadurch gekennzeichnet, daß** das Zahnradgetriebe aus wenigstens zwei miteinander in Eingriff stehenden Kegelrädern (6) besteht, wobei ein Kegelrad (6) mit dem distalseitigen Ende des inneren Rohres (4) verbunden ist und ein anderes Kegelrad (6) mit dem proximalseitigen Ende des distalen Teils (2) verbunden ist.

4. Drehgelenk nach Anspruch 3, **dadurch gekennzeichnet, daß** die beiden mit dem proximalen (1) und dem distalen Teil (2) verbundenen Kegelräder (6) über ein im Zwischenteil (3) gelagertes Kegelrad (12) miteinander verbunden sind.

5. Drehgelenk nach mindestens einem der Ansprüche 1 bis 4, **dadurch gekennzeichnet, daß** sich der maximale Auslenkungswinkel zwischen der Ausgangsstellung und der Endstellung aus der Gleichung

$$4 \times (90° - \alpha)$$

ergibt.

**Claims**

1. Rotary joint, in particular for medical instruments, having a proximal part (1) and a distal part (2), which are coupled to one another via a geared transmission and can be adjusted with respect to one another between an undeflected starting position and an angled-off end position, the geared transmission being mounted in an intermediate part (3) which is arranged between the proximal part (1) and the distal part (2), spaces these parts (1 and 2) apart from one another and is trapezium-shaped in longitudinal section, those end faces of the distal-side end (1a) of the proximal part (1) and of the proximal-side end (2a) of the distal part (2) which face one another and, in the starting position, bear against inclined end faces (3a) of the trapezium-shaped intermediate part (3) being inclined towards one another at the same angle (α) with respect to the longitudinal axis, and all three parts (1, 2 and 3) having at least one continuous hollow central passage (11), **characterized in that** the proximal part (1) comprises two tubes (4, 5) which are arranged coaxially inside one another and can rotate, independently of one another, about their longitudinal axis with respect to one another, at least the outer tube (5) being connected, on the proximal side, to a handle, and a control pin (9), which engages in a corresponding recess (10) in the intermediate part (3), being arranged on the distal-side end face (1a) of the outer tube (5).

2. Rotary joint according to Claim 1, **characterized in that** the inner tube (4) is provided on the distal side with a gearwheel of the geared transmission.

3. Rotary joint according to Claim 1 or 2, **characterized in that** the geared transmission comprises at least two intermeshing bevel gears (6), one bevel gear (6) being connected to the distal-side end of the inner tube (4) and another bevel gear (6) being connected to the proximal-side end of the distal part (2).

4. Rotary joint according to Claim 3, **characterized in that** the two bevel gears (6) which are connected to the proximal part (1) and the distal part (2) are connected to one another via a bevel gear (12) mounted in the intermediate part (3).

5. Rotary joint according to at least one of Claims 1 to 4, **characterized in that** the maximum angle of deflection between the starting position and the end position results from the equation

$$4 \times (90° - \alpha).$$

**Revendications**

1. Articulation tournante, en particulier pour instruments médicaux, comportant une partie proximale (1) et une partie distale (2) qui sont accouplées l'une à l'autre via un mécanisme à engrenages et déplaçables l'une par rapport à l'autre entre une position initiale non déviée et une position finale coudée, le mécanisme à engrenages étant monté dans une partie intermédiaire (3) de forme trapézoïdale en coupe longitudinale et agencée entre la partie proximale (1) et la partie distale (2) et écartant ces parties (1 et 2) l'une de l'autre, dans laquelle les surfaces frontales de l'extrémité (1a) côté distal de la partie proximale (1) et de l'extrémité (2a) côté proximal de la partie distale (2), tournées l'une vers l'autre et prenant dans la position initiale appui contre des surfaces frontales inclinées (3a) de la partie intermédiaire trapézoïdale (3), sont inclinées l'une vers l'autre sous le même angle (α) par rapport à l'axe longitudinal, et toutes les trois parties (1, 2 et 3) présentent au moins un canal central creux continu (11),

**caractérisée en ce que**

la partie proximale (1) est constituée par deux tubes (4, 5) agencés coaxialement l'un dans l'autre et mobiles en rotation l'un par rapport à l'autre autour de leur axe longitudinal indépendamment l'un de l'autre, le tube extérieur (5) au moins étant relié du côté proximal à une manette et un tenon de commande (9) étant agencé sur la surface frontale (1a) côté distal du tube extérieur (5), tenon qui s'engage dans un évidement correspondant (10) de la partie intermédiaire (3).

2. Articulation tournante selon la revendication 1, **caractérisée en ce que** le tube intérieur (4) est relié du côté distal à un engrenage du mécanisme à engrenages.

3. Articulation tournante selon l'une ou l'autre des revendications 1 et 2, **caractérisée en ce que** le mécanisme à engrenages est constitué par au moins deux engrenages coniques (6) en engrènement l'un avec l'autre, un engrenage conique (6) étant relié à l'extrémité côté distal du tube intérieur (4) et un autre engrenage conique (6) étant relié à l'extrémité côté proximal de la partie distale (2).

4. Articulation tournante selon la revendication 3, **caractérisée en ce que** les deux engrenages coniques (6) reliés à la partie proximale (1) et à la partie distale (2) sont reliés l'un à l'autre via un engrenage conique (12) monté dans la partie intermédiaire (3).

5. Articulation tournante selon l'une au moins des revendications 1 à 4, **caractérisée en ce que** l'angle de déviation maximal entre la position initiale et la position finale résulte de l'équation

$$4 \times (90° - \alpha).$$

Fig. 1

Fig. 2

Fig. 3